COMPLETE DOCUMENT

(19) Europäisches Patentamt
European Patent Office
Office européen des brevets

(11) Publication number: 0 113 566
A1

(12) # EUROPEAN PATENT APPLICATION

(21) Application number: 83307659.9

(22) Date of filing: 15.12.83

(51) Int. Cl.[3]: C 07 C 67/347
C 07 C 69/95

(30) Priority: 20.12.82 JP 222174/82

(43) Date of publication of application:
18.07.84 Bulletin 84/29

(84) Designated Contracting States:
DE FR GB IT

(71) Applicant: MEIJI SEIKA KAISHA LTD.
4-16 Kyobashi 2-chome
Chuo-ku Tokyo 104(JP)

(72) Inventor: Kishi, Yoshito
42 Tyler Road
Belmond Massachusetts(US)

(74) Representative: Marsh, Roy David et al,
Brewer & Son 5-9, Quality Court Chancery Lane
London WC2A 1HT(GB)

(54) **New intermediate compounds for synthesis of aklavinones and their preparation.**

(57) A new compound of the formula

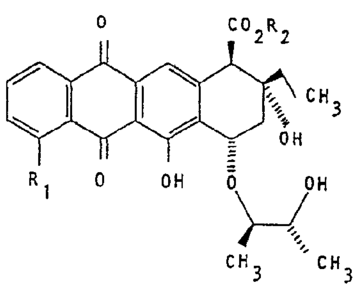

(I)

wherein $R_1$ is a hydrogen atom, a hydroxyl group or an alkoxyl group of 1-4 carbon atoms and $R_2$ is an alkyl group of 1-4 carbon atoms is now provided. This new compound is useful as intermediate for synthesis of aklavinones which may be coupled with sugars, for example, daunosamine to give anthracycline antibiotics. The new compound of this invention is produced by several reaction steps started from a 3-halo-5-substituted or unsubstituted naphthoquinone and a furan diene compound. This process involves new regio-specific Diels-Alder condensation and new asymmetric crossed aldol condensation.

EP 0 113 566 A1

# NEW INTERMEDIATE COMPOUNDS FOR SYNTHESIS OF AKLAVINONES AND THEIR PREPARATION

This invention relates to a new compound which is useful as intermediates for synthesis of aklavinones. This invention also relates to a process for the production of this new compound.

It is well known that aclacinomycin can be produced from aklavinone by enzymatic glycosidation (Journal of Antibiotics. Vol. 33, 1331 (1980)).

The present inventor has successfully synthesized a new compound of the formula (I)

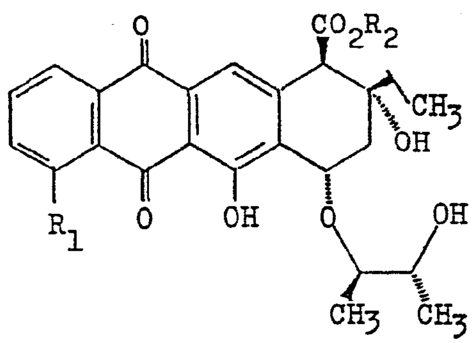

(I)

wherein $R_1$ is a hydrogen atom, a hydroxyl group or an alkoxyl group of 1-4 carbon atoms and $R_2$ is an alkyl group of 1-4 carbon atoms, started from a 3-bromo- or 3-chloro-5-substituted or unsubstituted naphthoquinone of the formula (II)

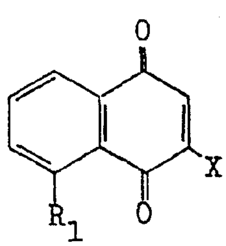

(II)

wherein X is a bromine or chlorine atom and $R_1$ is as defined above and a furan diene of the formula (III)

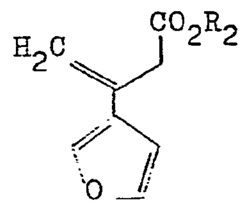

(III)

wherein $R_2$ is as defined above.

According to a first aspect of this invention, therefore, there is provided a new intermediate compound of the general formula (I)

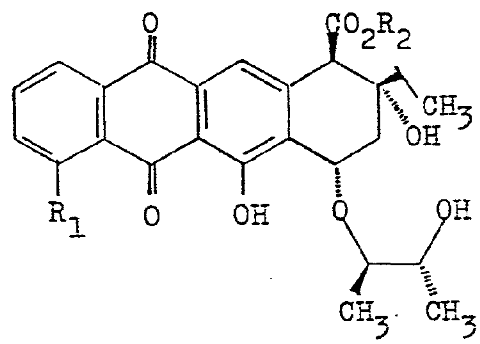

(I)

wherein $R_1$ denotes a hydrogen atom, a hydroxyl group of an alkoxyl group of 1-4 carbon atoms, particularly methoxy group and $R_2$ denotes an alkyl group of 1-4 carbon atoms.

According to a second aspect of this invention, there is provided a process for the production of a compound of the formula (I)

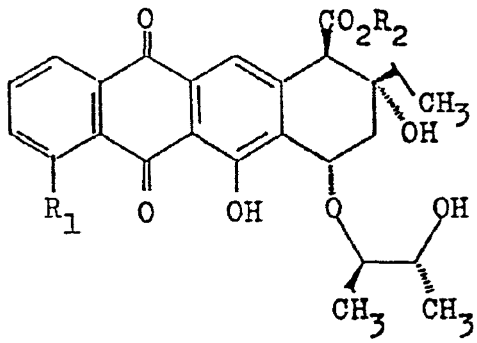

(I)

wherein $R_1$ denotes a hydrogen atom, a hydroxyl group or an alkoxyl group of 1-4 carbon atom and $R_2$ denotes an alkyl group of 1-4 carbon atoms, which comprises the steps of:-

(a) subjecting a compound of the formula (II)

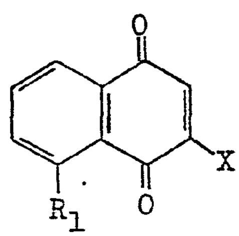

(II)

wherein $R_1$ is as defined above and X is a bromo or chloro group and a diene compound of the formula (III)

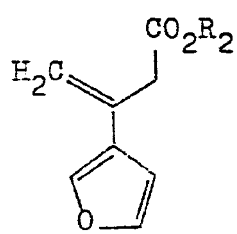

(III)

wherein $R_2$ is as defined above to regiospecific Diels-Alder condensation to produce the adduct of the formula (IV)

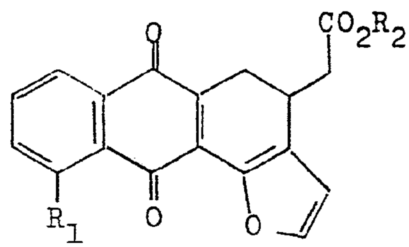

(IV)

wherein $R_1$ and $R_2$ are as defined above,

(b) oxidizing the adduct of the formula (IV) to produce the compound of the formula (V)

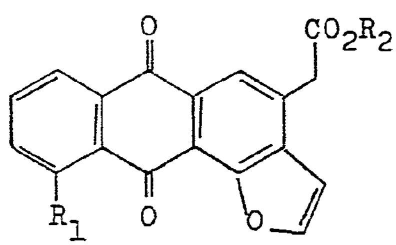

(V)

wherein $R_1$ and $R_2$ are as defined above,

(c) subjecting the compound of the formula (V) to ozonolysis to produce an aldehyde compound of the formula (VI)

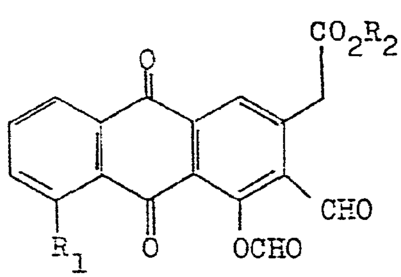

(VI)

wherein $R_1$ and $R_2$ are as defined above,

(d) acetalyzing the aldehyde compound of the

formula (VI) with D-(-)-2,3-butanediol to produce a compound of the formula (VII)

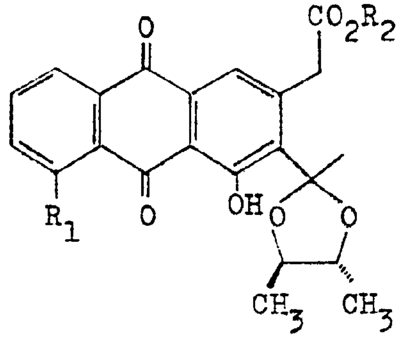

(VII)

wherein $R_1$ and $R_2$ are as defined above,

(e) subjecting the compound of the formula (VII) and 1-(trimethylsilyl)-2-butanone to asymmetric crossed aldol condensation to produce a compound of the formula (VIII)

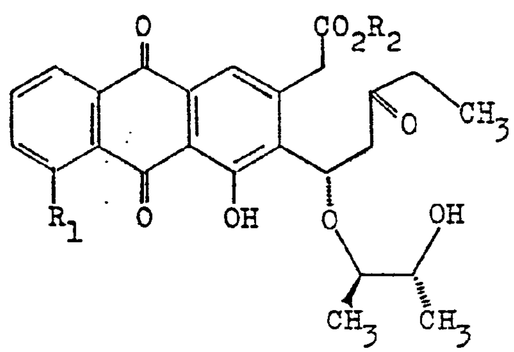

(VIII)

wherein $R_1$ and $R_2$ are as defined above, and

(f) cyclizing the compound of the formula (VIII) to produce the desired compound of the formula (I).

In the process of this invention, Diels-Alder reaction of the naphthoquinone or juglone of the formula

(II) and the furan diene of the formula (III) occurs regiospecifically to give the desired adduct of the formula (IV). Furthermore, crossed aldol condensation of the acetal compound of the formula (VII) and the silyl ketone, for example, 1-(trimethylsilyl)-2-butanone, gives higher asymmetric induction.

These new methods discovered by the present inventor establish the asymmetric synthesis of anthracyclinones.

The steps of the process of this invention are depicted by the following reaction scheme:-

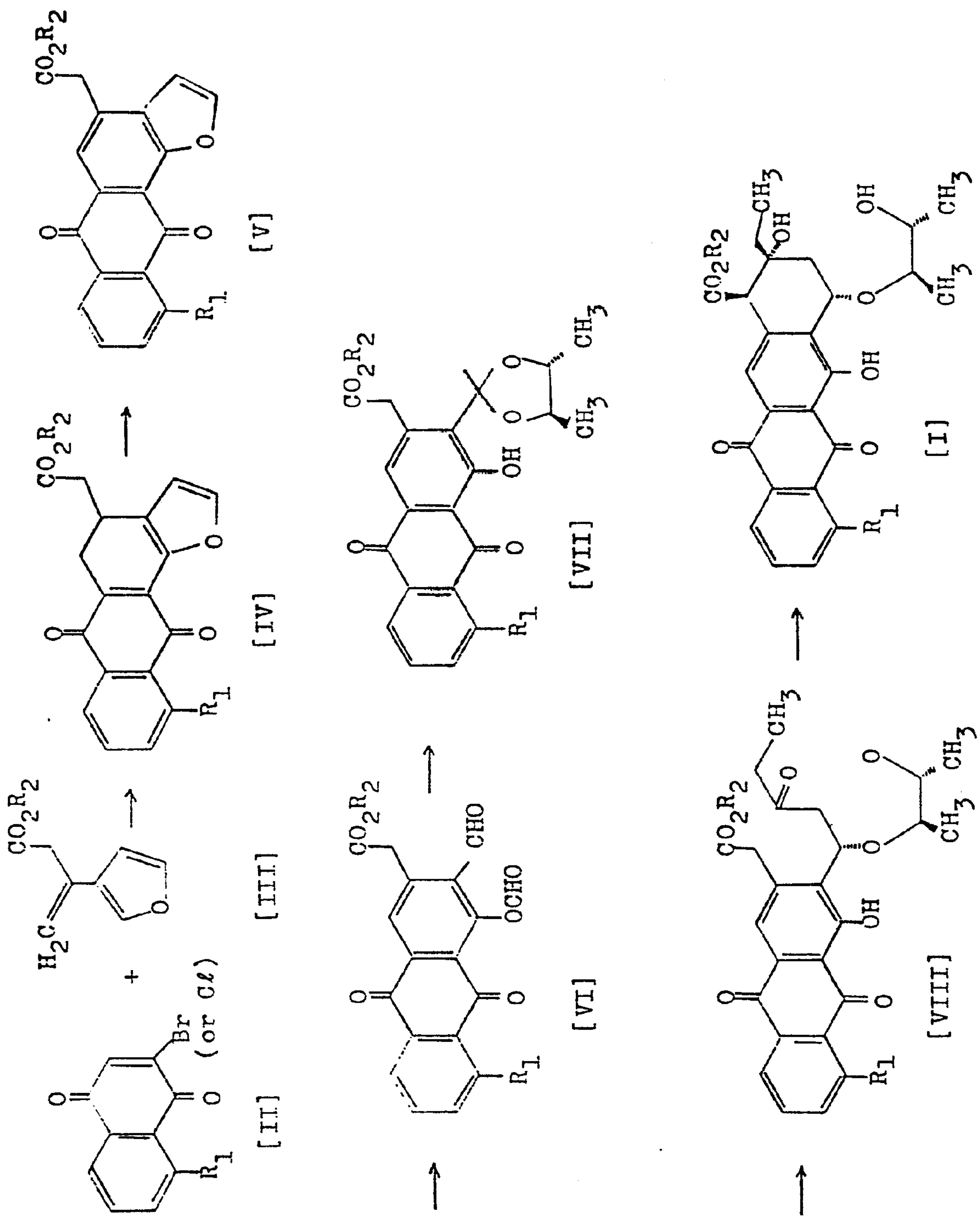

[II] [III] [IV] [V]

[VI] [VII]

[VIII] [I]

In the above reaction scheme, $R_1$ denotes a hydrogen atom, a hydroxyl group or an alkoxyl group such as methoxy group and $R_2$ denotes an alkyl group of 1-4 carbon atoms.

The process of this invention is now described in detail in respect of the steps of the process.

In the first step, the Diels-Alder condensation of the dienophile compound (II) with the diene compound (III) is carried out preferably under heating at a refluxing temperature of an inert organic solvent such as aromatic hydrocarbons, for example, benzene, toluene, xylene. In order to prevent degradation of the adduct product (IV), it is preferred to use 4,4-thiobis(6-t-butyl-3-methylphenol) as a radical scavenger and also strontium carbonate ($SrCO_3$) to neutralize the hydrogen halide that is formed during the reaction. After the Diels-Alder reaction, the adduct of the formula (IV) can be used for next step without purification. Thus, in the second step, the crude adduct product in chloroform solution may be air-oxidized in the presence of a base such as an alkylamine, preferably di-isopropylamine to afford the compound of the formula (V).

While, if the Diels-Alder condensation of the compound (II) with the compound (III) is carried out under acidic conditions followed by air-oxidation, a compound of the formula (V') given below, that is, a regioisomer of the compound (V), is formed selectively.

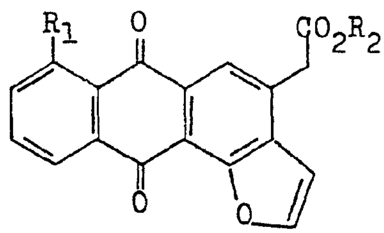

(V')

In the second, oxidation step, the air-oxidation of the compound (IV) may conveniently be effected by stirring a solution of the compound (IV) in dry chloroform under air atmosphere at ambient temperature or a slightly elevated temperature.

Subsequently, in the third step, the compound (V) is ozonolyzed to produce the aldehyde compound (VI). Low temperature, particularly a temperature in the vicinity of -78°C is preferred in this ozonolysis reaction. A halogenated hydrocarbon such as dichloromethane, chloroform and carbon tetrachloride can be used suitably as the solvent.

Then, in the fourth step, the aldehyde compound (VI) is reacted with D-(-)-2,3-butanediol in the presence of an acid catalyst such as pyridinium p-toluenesulfonate or camphor sulfonic acid. The reaction of this acetalization step may conveniently be effected in dry benzene at the reflux temperature of the solvent under argon atmosphere.

In the further step, the acetal compound (VII) is reacted with 1-(trimethylsilyl)-2-butanone at a low temperature, preferably at -23° to 10°C, to give the compound

(VIII) of the formula (VIII) as a main product, together with its stereo isomer of the formula (VIII')

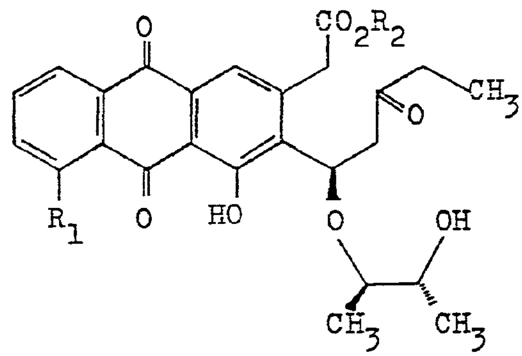

(VIII')

wherein $R_1$ and $R_2$ are as defined hereinbefore.

This asymmetric crossed aldol condensation may be carried out in an anhydrous organic solvent, preferably dry acetonitrile or dry tetrahydrofuran and in the presence of an acid catalyst such as a Lewis acid, especially titanium tetrachloride or tin tetrachloride.

In the final, cyclization step of the present process, the compound (VIII) is ring-closed by treating with a base such as an alkali metal carbonate, preferably potassium carbonate in an organic solvent such as methanol at ambient temperature to afford the desired compound (I) of this invention, together with its stereo isomer of the formula (I')

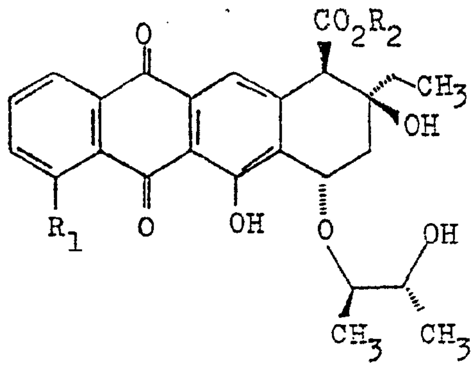

(I')

Recovery of the compound (I) from the reaction mixture containining both the compounds (I) and (I') may be made by acidifying the reaction mixture with 1 N hydrochloric acid, admixing the acidified reaction mixture with dichloromethane and water, washing the aqueous layer with dichloromethane, washing the combined organic extracts with water and drying over anhydrous sodium sulfate, followed by removing the solvent from the organic extracts under reduced pressure to give a crude product comprising both the compounds (I) and (I'). This crude product may be purified by preparative thin layer chromatography on silica gel developed with 12% ethyl acetate in dichloromethane to obtain the compound (I) and the compound (I') separately.

The compound (I) of this invention can be converted into aklavinone of the formula (IX)

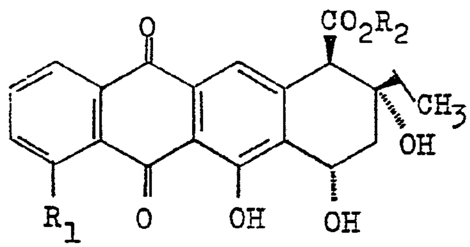

(IX)

wherein $R_1$ and $R_2$ are as defined above, for example, by treatment with trifluoroacetic acid at -78° to 10°C under argon atmosphere.

Example 1

(a) Compound of the formula (Va) was prepared according to the following route:

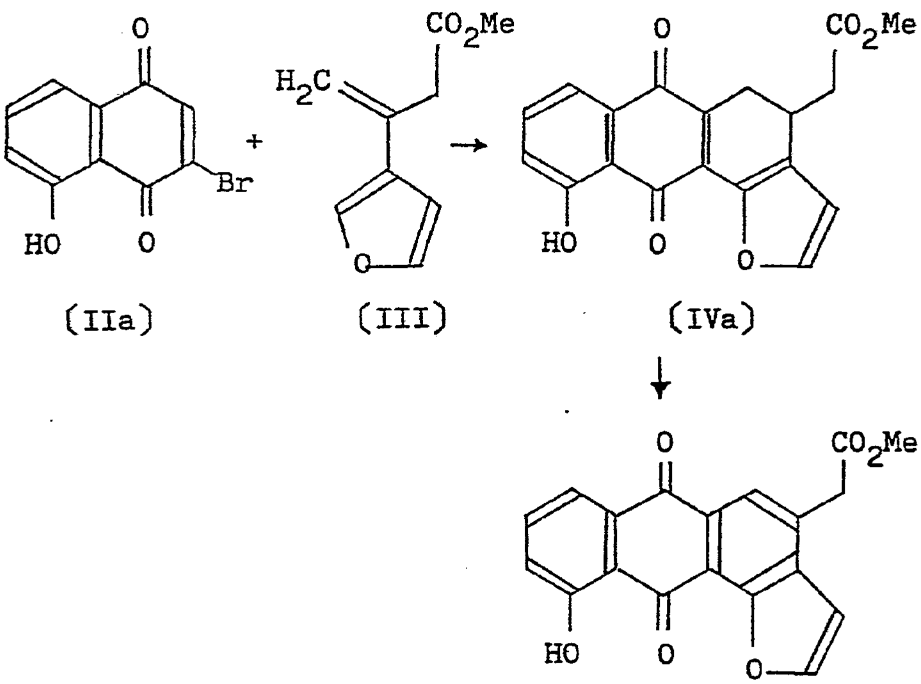

(IIa) (III) (IVa)

(Va)

where Me denotes a methyl group.

A 50 m$\ell$ round bottom flask was charged with 0.201 g (0.794 mmoles) of compound (IIa), compound (III) (equivalent amount of compound (IIa)), 0.395 g (2.38 mmole, 3.0 equiv.) of $SrCO_3$, and approx. 5 mg of the radical scavenger, 4,4'-thiobis(6-t-butyl-3-methylphenol). The flask was flushed with argon and 20 m$\ell$ of dry benzene was added. The reaction mixture was refluxed for 19 hrs., cooled

to room temperature and the inorganic salts were removed by filtration.

After the solvent was removed in vacuo, the residue was purified by ptlc. (preparative thin layer chromatography) (silica; 20 cm x 20 cm x 2 mm; 30% ethyl acetate (EtOAc) in hexane as a eluant) to afford 0.203 g (0.601 mmole; 75.6%) of compound (IVa) (dark red color) and 19.3 mg of compound (IIa) (unreacted) (the corrected yield of compound (IVa) is 84%).

Compound (IVa) was then dissolved in 5mℓ of $CHCℓ_3$ and 100 μℓ of diisopropylethylamine. The solution was stirred under an air atmosphere for 12 hrs. The solvent was removed in vacuo to give a blackish solid (NMR shows this compound is of good purity). The blackish solid was passed through a short silica column ($CH_2Cℓ_2$, then 30% EtOAc/hexane, then 50% EtOAc/hexane as eluant) to afford yellow crystals (mp 209-211°C) of compound (Va) in nearly quantitative yield.

NMR ($CDCℓ_3$): δ 3.72 ppm (3H, s), 3.99 (2H, s), 6.98 (1H, d, J = 2.2 Hz), 8.04 (1H, d, J = 2.2 Hz), 8.11 (1H, s), 12.60 (1H, s).

---

IR (KBr): ν 1735 $cm^{-1}$, 1665, 1635, 1590, 1570

---

UV (MeOH): 226 nm (log ε = 4.57), 273 (4.54), 295 (sh. 4.13), 391 (4.09)

(b) Compound (VIIa) was prepared according to the following route:

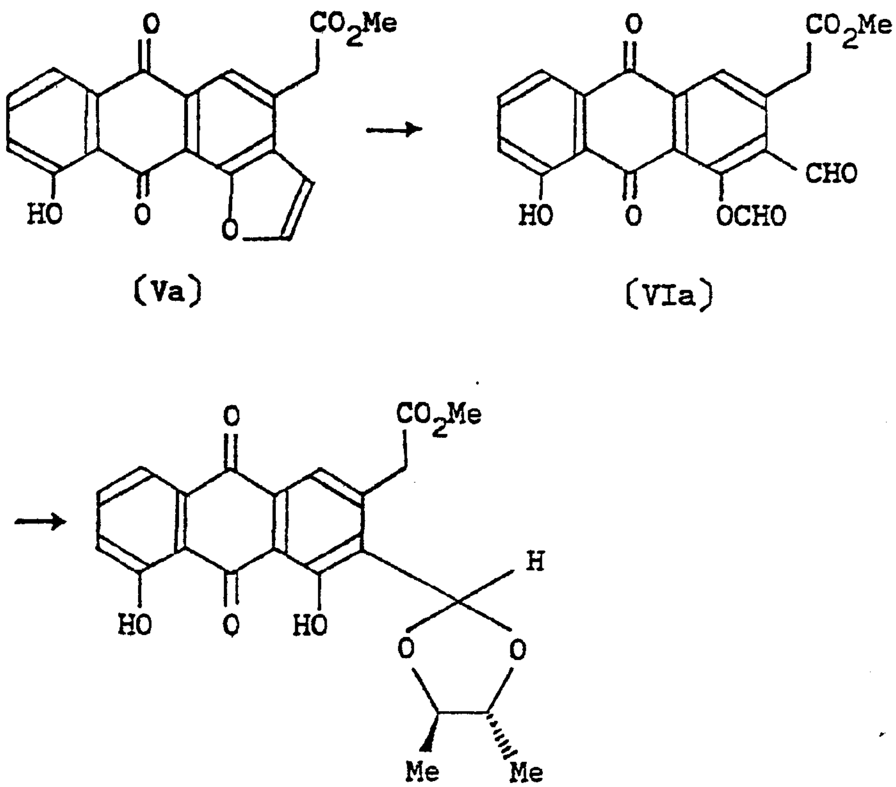

(Va)

(VIa)

(VIIa)

The compound (Va) (0.560 g, 1.66 mmol) was dissolved in 450 m$\ell$ of $CH_2C\ell_2$ in a 1 $\ell$ flask with a $CaSO_4$ drying tube and cooled to -78°C. Ozone was bubbled through the solution for 12 min. with stirring. Excess ozone was then removed by bubbling argon through the solution for 45 minutes at -78°C. Dimethyl sulfide (30 m$\ell$) was added with stirring, and the cooling bath was removed. After one hour, the reaction mixture was concentrated at

reduced pressure to leave ca. 0.70 g of crude aldehyde compound (VIa) which was contaminated with dimethyl sulfoxide.

Crude compound (VIa) was dried by azeotropic evaporation with toluene and then suspended in 50 m$\ell$ of dry benzene in a 200 m$\ell$ flask. D-(-)-2,3-Butanediol (0.75 g, 8.3 mmol) and pyridinium p-toluenesulfonate (ca 20 mg, 0.08 mmol) were added and the reaction mixture was refluxed with azeotropic removal of water under argon for 24 hrs. After cooling to room temperature, the reaction mixture was partitioned between $CH_2C\ell_2$ and 5% aqueous $NaHCO_3$. The aqueous layer was extracted with $CH_2C\ell_2$ and the combined organic extracts were washed with two portions of water and dried over $NaSO_4$. Solvent was removed under reduced pressure to give the crude product which was purified by chromatography on silica gel (100 g) with elution by $CH_2C\ell_2$ - 5% EtOAc/$CH_2C\ell_2$. The acetal compound (VIIa) (0.632 g. 1.53 mmol; 92.0%) of pure state was obtained.

An analytical sample was prepared by twice recrystallizing the product from hot methanol (mp 192~194°C; $(\alpha)_D$ -47.9° (c 0.19, $CHC\ell_3$)).

(c) Compound (VIIIa) was prepared according to the following route:

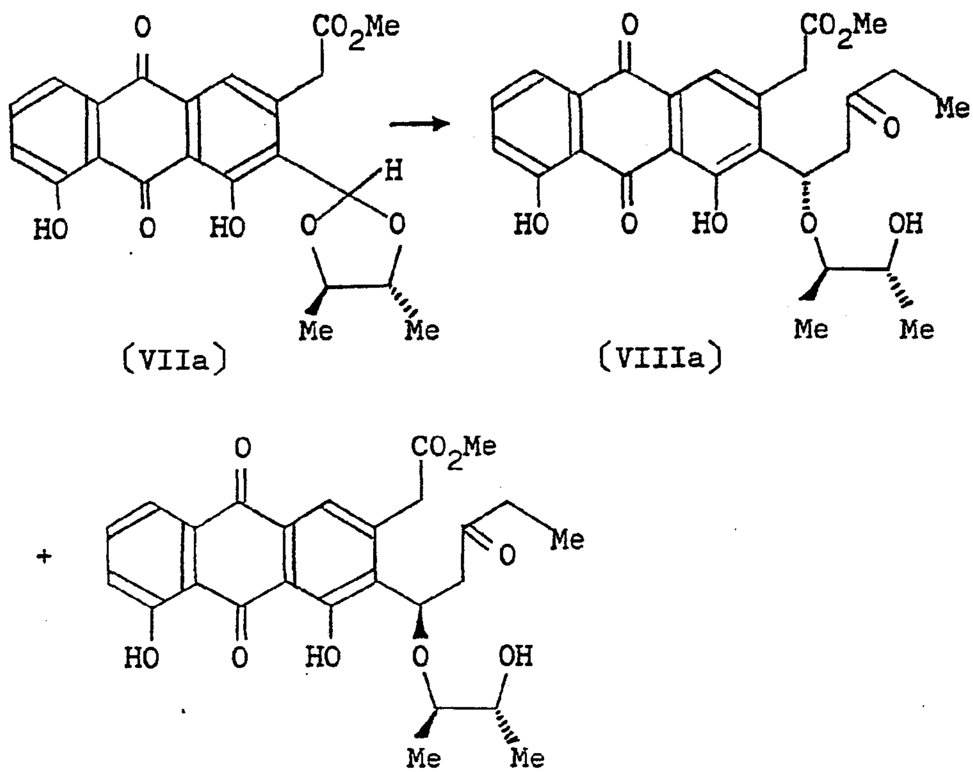

(VIIa) (VIIIa)

+

(VIII'a)

The acetal compound (VIIa) (288 mg, 0.698 mmol), 1-(trimethylsilyl)-2-butanone ($TMSCH_2COCH_2CH_3$) (1.41 g, 9.78 mmol) and dry $CH_3CN$ (105 m$\ell$) were placed in a dry 250 m$\ell$ flask under argon. The reaction mixture was cooled to ca -23°C and 0.95 m$\ell$ (1.04 mmol) of a solution of $SnC\ell_4$ in $CH_2C\ell_2$ (1.1 M, Alfa) was added dropwise with stirring over three minutes. After three hours at ca -23°C, the temperature of the cooling bath was allowed

to slowly rise to -10°C. Saturated aqueous $NaHCO_3$ (50 m$\ell$) was added (the total reaction time was 4.5 hrs.) and the reaction mixture was partitioned between $CH_2C\ell_2$ and water. The aqueous layer was extracted with three portions of $CH_2C\ell_2$ and the combined organic extracts were dried over $Na_2SO_4$. Solvent was removed under reduced pressure to give the crude product, which was dissolved in warm benzene. Hexane was added and after gradual cooling to -15°C, the major crossed aldol condensation product (VIIIa) crystallized. The crude product was collected by filtration, washed with hexane, and recrystallized from benzene/hexane to afford 157 mg (0.324 mmol) of pure compound (VIIIa). The combined mother liquors were purified by ptlc. on silica gel with 12% EtOAc in benzene as eluant to give an additional 98 mg (0.202 mmol; total yield = 75.3%) of compound (VIIIa) and 25.4 mg (0.0524 mmol, 7.5% yield) of the stereo isomer (VIII'a) (TLC:Rf of major product (VIIIa): 0.36; Rf of minor product (VIII'a): 0.27; 15% EtOAc/$CH_2C\ell_2$).

An analytical sample of compound (VIIIa) was prepared by twice recrystallizing the product from benzene/hexane (mp 120-124°C, $(\alpha)_D$ -155° (c 0.19, $CHC\ell_3$)).

(d) Compound (Ia), one example of the new compound of this invention, was prepared according to the following route:

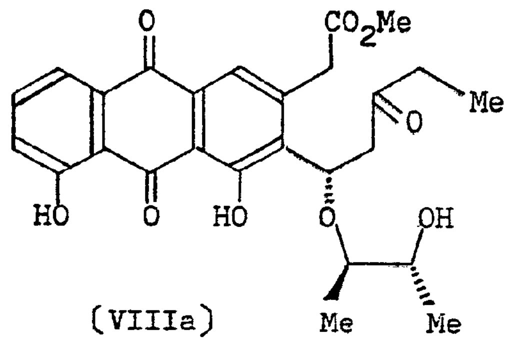

(VIIIa)

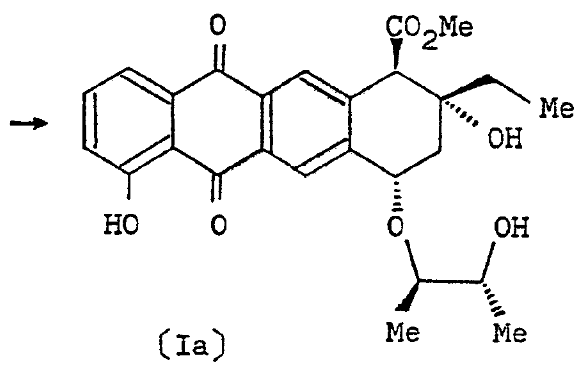

(Ia)

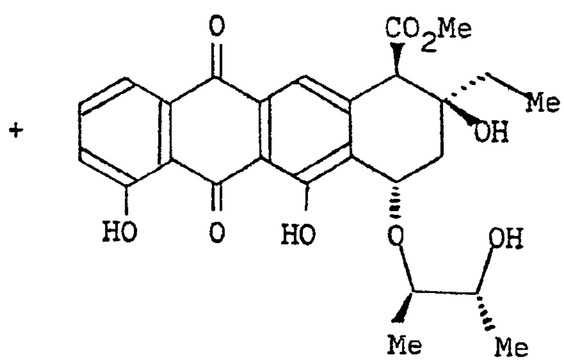

(I'a)

To a stirred suspension of 179 mg (0.3695 mmol) of Compound (VIIIa) in 60 mℓ of methanol was added 124 mg (0.897 mmol) of $K_2CO_3$. After stirring the resultant blood red solution for 2.5 hrs. at room temperature, two mℓ of 1N aqueous HCℓ was added. The reaction mixture was partitioned between $CH_2Cℓ_2$ and water and the aqueous layer was extracted with $CH_2Cℓ_2$. The combined organic extracts were washed with water and dried over $Na_2SO_4$. Solvent was removed under reduced pressure to give the crude product, which was purified by ptlc. on silica gel with 12% EtOAc in $CH_2Cℓ_2$ as eluant. 95.6 mg (0.197 mmol, 53% yield) of compound (Ia) and 74.5 mg (0.154 mmol, 42% yield) of compound (I'a) were obtained (TLC:Rf of compound (Ia): 0.57; Rf of compound (I'a): 0.25; 15% EtOAc/$CH_2Cℓ_2$ x 2).

An analytical sample of compound (Ia) was prepared by twice recrystallizing the product from $Et_2O$/hexane (mp 116°C, then 163-165°C (double melting point); $(\alpha)_D$ + 202° (c 0.38, $CHCℓ_3$)).

An analytical sample of compound (I'a) was prepared by twice recrystallizing the product from $Et_2O$/hexane (mp 178-180°C, $(\alpha)_D$ + 180° (c 0.15, $CHCℓ_3$)).

Example 2

This Example illustrates the production of aklavinone from compound (Ia) obtained in Example 1.

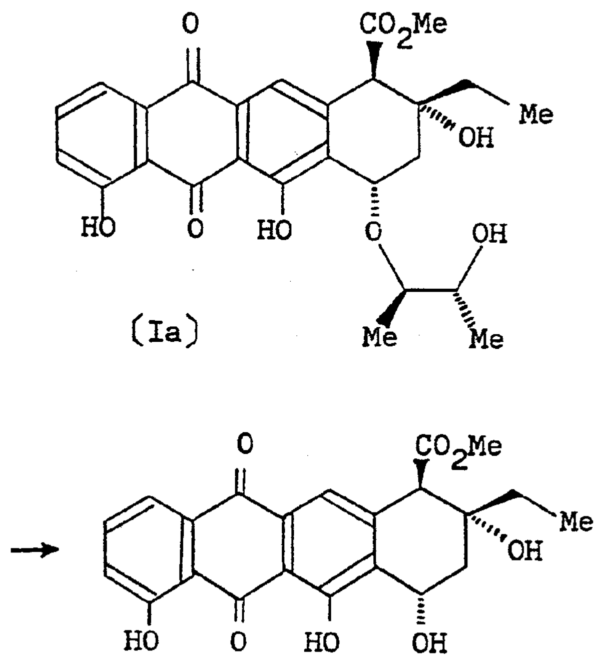

(Ia)

(IXa)

The ether compound (Ia) (43.6 mg, 0.090 mmol) and a magnetic stirring bar were placed in a 50 mℓ flask under argon and cooled to -78°C. Three mℓ of trifluoroacetic acid was added dropwise down the sides of the flask over two minutes. The temperature of the cooling bath was allowed to slowly rise to 10°C over three hours; stirring was begun when the trifluoroacetic acid began

to melt at ca -15°C. After the three hour period, the cooling bath was removed and stirring was continued for 1.7 hours. The reaction mixture was then thoroughly concentrated at reduced pressure, and the crude product was dissolved in 7.2 mℓ of acetone. Three mℓ of freshly prepared 5% aqueous $NaHCO_3$ was added with stirring, and after one hour 16 mℓ of water was added. The mixture was extracted three times with $CH_2Cℓ_2$ and the combined organic extracts were dried over $Na_2SO_4$ and concentrated at reduced pressure. The crude product was purified by ptlc. on silica gel with 12% EtOAc in benzene as eluant (two developments) to afford 31.3 mg (0.0760 mmol, 84% yield) of pure (+)-aklavinone of the formula (IXa). A sample was crystallized from hot benzene to give red-orange needles; mp 170-172°C, $(\alpha)_D$ + 150° (c 0.214, $CHCℓ_3$).

Example 3

This Example illustrates that when the step of Diels-Alder reaction involved in the process of this invention is carried out under an acidic condition, there is selectively afforded a regioisomer of the formula (V'a) in which the position of the substituent $R_1$ (e.g. hydroxyl) is displaced from that of the compound of the formula (Va).

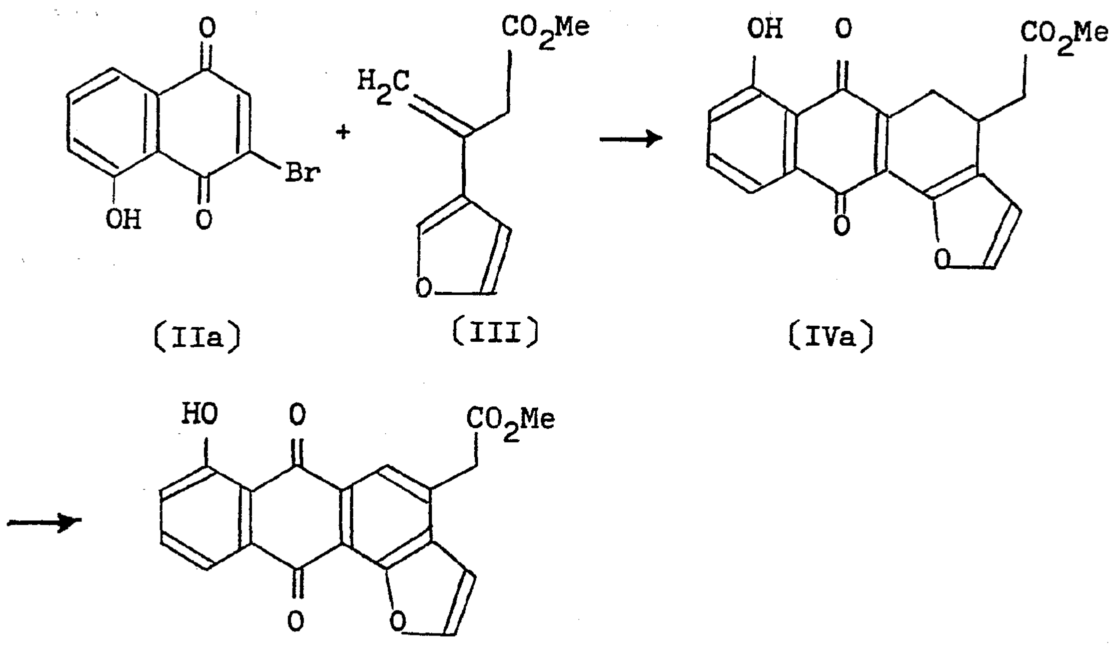

(IIa) (III) (IVa)

(V'a)

To a solution of 50.6 mg (0.20 mmol) of the bromojuglone compound (IIa) in 20 m$\ell$ of dry $CH_3CN$ cooled to -20°C under nitrogen with stirring was introduced excess gaseous $BF_3$. Furan diene compound (III) (66 mg, 0.30 mmol) was added in one portion, and after stirring for ten minutes at -20°C, 15 m$\ell$ of 20% aqueous sodium acetate (NaOAc) was added and the cooling bath was removed. The mixture was stirred at room temperature for 30 minutes and then heated to 50°C for five minutes. After cooling to room temperature, 20 m$\ell$ of ethyl acetate (EtOAc) was added and the layers separated. The organic layer was washed with saturated aqueous $NaHCO_3$, saturated aqueous NaC$\ell$, and dried over $Na_2SO_4$. Solvent was removed

under reduced pressure and the crude product was purified by silica gel TLC with 5% EtOAc in benzene as eluant to give 27.6 mg (0.081 mmol, 41% yield) of compound (IVa) and 6.2 mg (0.018 mmol, 9% yield) of compound (V'a). Compound (IVa) was converted to compound (V'a) under the usual conditions, i.e. air-oxidation in $CHCl_3$ containing diisopropylethylamine at room temperature.

NMR ($CDCl_3$): $\delta$ 3.73 ppm (3H, s), 6.98 (1H, d, J = 2.2 Hz), 8.03 (1H, d, J = 2.2 Hz), 8.14 (1H, s), 12.59 (1H, s)

Compound (V'a) so prepared may be used for synthesis of a regioisomer of anthracyclinones.

CLAIMS

1. A compound of the formula (I)

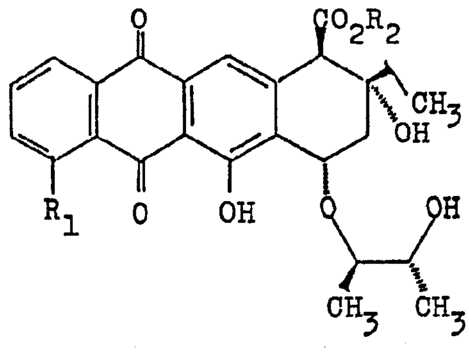

(I)

wherein $R_1$ is a hydrogen atom, a hydroxyl group or an alkoxyl group of 1-4 carbon atoms and $R_2$ is an alkyl group of 1-4 carbon atoms.

2. The compound of Claim 1 which is the compound of the formula (I) where $R_1$ is a hydroxyl group and $R_2$ is a methyl group.

 

3. A process for the production of a compound of the formula (I) as claimed in Claim 1, which comprises the steps of:-

(a) subjecting a compound of the formula (II)

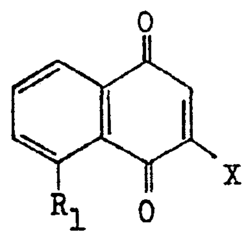

(II)

wherein $R_1$ is as defined above and X is a bromo or chloro group and a compound of the formula (III)

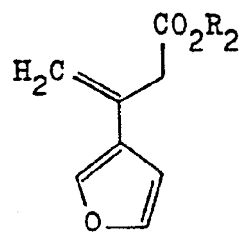

(III)

wherein $R_2$ is as defined above to Diels-Alder condensation to produce the adduct of the formula (IV)

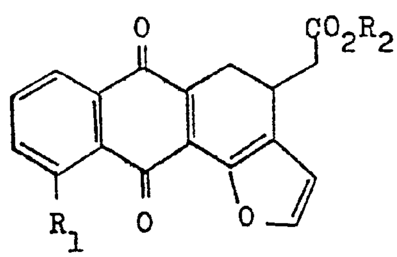

(IV)

wherein $R_1$ and $R_2$ are as defined above,

(b) oxidizing the adduct of the formula (IV) to produce the compound of the formula (V)

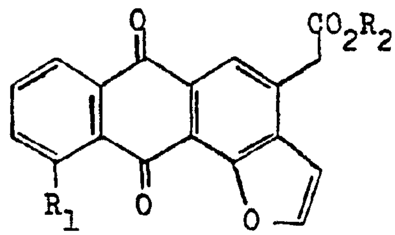

(V)

wherein $R_1$ and $R_2$ are as defined above,

(c) subjecting the compound of the formula (V) to ozonolysis to produce an aldehyde compound of the formula (VI)

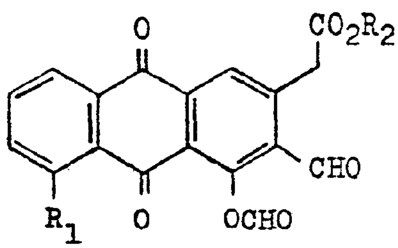

(VI)

wherein $R_1$ and $R_2$ are as defined above,

(d) acetalyzing the aldehyde compound of the formula (VI) with D-(-)-2,3-butanediol to produce a compound of the formula (VII)

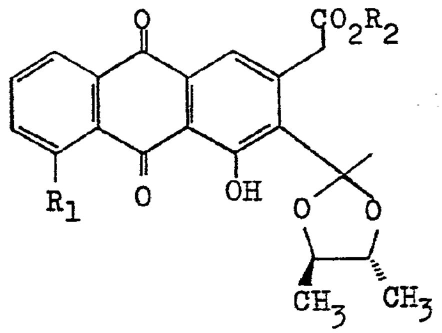

(VII)

wherein $R_1$ and $R_2$ are as defined above,

(e) subjecting the compound of the formula (VII) and 1-(trimethylsilyl)-2-butanone to asymmetric crossed aldol condensation to produce a compound of the formula (VIII)

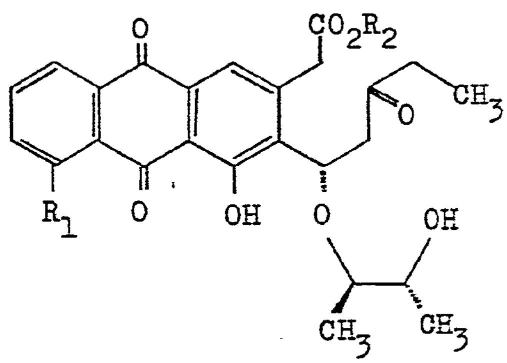

(VIII)

wherein $R_1$ and $R_2$ are as defined above, and

(f) cyclizing the compound of the formula (VIII) to produce the desired compound of the formula (I).

4. A process as claimed in Claim 3 in which the compound of the formula (II) is condensed with the compound of the formula (III) under thermal Diels-Alder reaction conditions in an aromatic hydrocarbon in the presence of 4,4'-thiobis (6-t-butyl-3-methylphenol) as a radical scavenger and strontium carbonate as a neutralizing agent.

5. A process as claimed in Claim 3 in which the compound of the formula (VII) and 1-(trimethylsilyl)-2-butanone are subjected to asymmetric crossed aldol condensation at a temperature of -23°C to 10°C in the presence of a Lewis acid as the catalyst.

European Patent Office

# EUROPEAN SEARCH REPORT

0113566

Application number

EP 83 30 7659

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X,Y | JOURNAL OF THE AMERICAN CHEMICAL SOCIETY, vol. 104, no. 25, 15th December 1982, pages 7371-7372, American Chemical Society J.M. McNAMARA et al.: "Practical asymmetric synthesis of aklavinone" * Pages 7371-7372 * --- | 1-5 | C 07 C 67/347<br>C 07 C 69/95 |
| Y | JOURNAL OF THE AMERICAN CHEMICAL SOCIETY, vol. 104, no. 25, 15th December 1982, pages 7372-7374, American Chemical Society H. SEKIZAKI et al.: "Practical asymmetric syntheses of 11-deoxydaunomycinone and related compounds" * Pages 7372-7374 * ----- | 1-5 | |
| | | | **TECHNICAL FIELDS SEARCHED (Int. Cl. 3)** |
| | | | C 07 C 67/00<br>C 07 C 69/00 |

The present search report has been drawn up for all claims

| Place of search | Date of completion of the search | Examiner |
|---|---|---|
| THE HAGUE | 02-04-1984 | VERHULST W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document